# EUROPÄISCHE PATENTSCHRIFT

(11) **EP 2 655 354 B1**
(45) Veröffentlichungstag und Bekanntmachung des Hinweises auf die Patenterteilung: **06.08.2014**
(21) Anmeldenummer: 11805419.6
(22) Anmeldetag: 21.12.2011
(51) Int. Cl.: C07D 311/92

(54) **PHOTOCHROME ANNELLIERTE NAPHTHOPYRANE MIT EINEM ZUM PYRANSAUERSTOFF BENACHBARTEN BENZOLRING, DER ÜBER BEIDE META-STELLUNGEN MIT DEM PARA-SUBSTITUENTEN VERKNÜPFT IST**
PHOTOCHROMIC ANELLATED NAPHTHOPYRANS HAVING A BENZENE RING WHICH IS ADJACENT TO THE PYRAN ACID AND IS LINKED TO THE PARA SUBSTITUENTS VIA BOTH META POSITIONS
NAPHTOPYRANE FUSIONNÉ PHOTOCHROME COMPORTANT UN NOYAU BENZÉNIQUE ADJACENT À UN OXYGÈNE DE PYRANE, LE NOYAU BENZÉNIQUE ÉTANT LIÉ PAR LES DEUX POSITIONS MÉTA AVEC LES SUBSTITUANTS EN PARA

(30) Priorität: 23.12.2010 DE 102010055760
(43) Veröffentlichungstag der Anmeldung: 30.10.2013
(73) Patentinhaber: Rodenstock GmbH, 80687 München (DE)
(72) Erfinder: MELZIG, Manfred, 82234 Wessling (DE); ROHLFING, Yven, 81547 München (DE); WEIGAND, Udo, 81247 München (DE)
(74) Vertreter: Müller-Boré & Partner Patentanwälte PartG mbB
(86) Internationale Anmeldenummer: PCT/EP2011/006479
(87) Internationale Veröffentlichungsnummer: WO 2012/084231

(56) Entgegenhaltungen:
- EP-A1- 1 184 379
- EP-A1- 1 394 156
- WO-A1-2009/024271
- WO-A1-2009/132842

## Beschreibung

Die vorliegende Erfindung betrifft photochrome annellierte Naphthopyrane mit einem zum Pyransauerstoff benachbarten Benzolring, der über beide meta-Stellungen mit dem para-Substituenten verknüpft ist, sowie deren Verwendung in Kunststoffen aller Art, insbesondere für ophthalmische Zwecke.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren zwei Pyran-Verbindungsklassen entwickelt werden, die für den Einsatz in phototropen Brillengläsern geeignet sind. Dabei handelt es sich um 2,2-Diaryl-2H-naphtho[1,2-b]pyrane sowie 3,3-Diaryl-3H-naphtho[2,1-b]pyrane, deren offene (angeregte) Formen verschiedene Eindunklungsfarben von gelb bis rotviolett aufweisen.

2,2-Diaryl-2H-naphtho[1,2-b]pyrane mit einer zusätzlichen Annellierung am pyranoannellierten Benzolring sind von großem Interesse, da sie aufgrund ihres größeren Ringsystems längerwellig absorbieren und somit violette bzw. blaue Eindunklungsfarben zugänglich sind. Bei dieser zusätzlichen Annellierung handelt es sich in der Regel um einen Benzolring (in Formel (I) der Benzolring mit den Substituenten R²), der nochmals in ortho-Stellung mit dem Naphthopyran verbrückt ist (siehe in Formel (I) Z).

Wird diese Verbrückung nur über ein Atom erzeugt, so ergibt sich ein an das Benzopyran annellierter Fünfring. Beispiele finden sich für ein Kohlenstoffatom in US 5,645,767, US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Die Substituenten in ortho-Stellung zum Pyran-Sauerstoffatom werden im Stand der Technik meist nur mit B und B' bezeichnet. Sie stellen meist aromatische oder heteroaromatische Substituenten dar. Zudem können die Substituenten B und B' zusammen ein eigenes cyclisches aliphatisches oder aromatisches oder heteroaromatisches Ringsystem bilden. Über eine Beeinflussung der photochromen Eigenschaften durch geeignete Auswahl der Substituenten B und B' wird - mit Ausnahme von ortho-Substituenten am Benzolring benachbart zum Pyransauerstoff - im Stand der Technik nahezu nichts berichtet. Selbst kleine ortho-Substituenten am Benzolring benachbart zum Pyran-Sauerstoff wie Fluor, Methyl oder Methoxy verlangsamen die Aufhellgeschwindigkeit so stark, dass sie für den beabsichtigten Verwendungszweck in Brillengläsern nicht verwendbar sind.

Der vorliegenden Erfindung liegt daher die Aufgabe zugrunde, eine Klasse neuer photochromer Verbindung bereitzustellen, die verbesserte Eigenschaften gegenüber den im Stand der Technik beschriebenen Strukturen aufweisen sollen. Dazu zählen unter anderem eine sehr hohe Eindunklungsleistung auch bei höheren Temperaturen, eine schnelle Aufhellgeschwindigkeit sowie eine sehr gute Lichtbeständigkeit.

Diese Aufgabe wird durch die in den Ansprüchen gekennzeichneten Gegenstände gelöst.

Insbesondere werden photochrome Naphthopyrane mit den allgemeinen Formeln (I) bzw. (II) bereitgestellt: 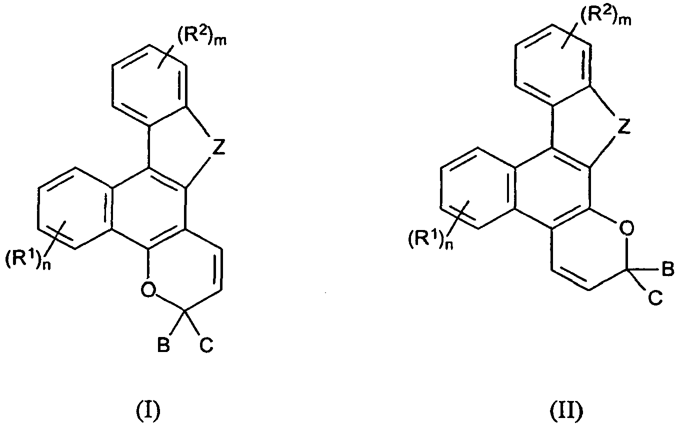 worin
die Reste R¹ und R² jeweils unabhängig voneinander einen Substituenten darstellen, ausgewählt aus der Gruppe α, bestehend aus einem Wasserstoffatom, einem (C₁ - C₆)-Alkylrest, einem (C₁ - C₆)-Thioalkylrest, einem (C₃ - C₇)-Cycloalkylrest, der ein oder mehrere Heteroatome, wie beispielsweise O oder S, aufweisen kann, einem(C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder zwei Reste R¹ und/oder zwei Reste R² jeweils unabhängig voneinander einen un-, mono- oder disubstituierten, an das Grundgerüst annellierten Benzo-, Pyrido-, Benzofuro- oder Benzothienoring bilden, dessen Substituenten aus der Gruppe α ausgewählt sein können;
Z aus -CR¹⁰R¹¹-, -O-, -S-, -NPh-, -N(C₁-C₆)Alkyl, -O-CR¹⁰R¹¹-,
-CR¹⁰R¹¹-O-, -S-CR¹⁰R¹¹-, -CR¹⁰R¹¹-S-, -CR¹⁰R¹¹-CR¹⁰R¹¹-, -CR¹⁰=N- oder -CR¹⁰=CR¹¹- ausgewählt ist, wobei die Substituenten R¹⁰ und R¹¹ aus der Gruppe α, vorzugsweise aus einem Wasserstoffatom, einem (C₁ - C₆)-Alkylrest, einem (C₃ - C₇)-Cycloalkylrest, einem un-, mono- oder disubstituiertem Phenyl-, Benzyl- oder Naphthylrest ausgewählt sein können;
oder Z unter Einbeziehen des Spiro-Kohlenstoffatoms einen 3- bis 8-gliedrigen carbo- oder heteromonozyklischen Ring darstellt, der gegebenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können;
oder Z unter Einbeziehen des Spiro-Kohlenstoffatoms einen 7- bis 12-gliedrigen carbo-bizyklischen Ring bzw. einen 7- bis 12-gliedrigen carbo-trizyklischen Ring darstellt, der wiederum einen, zwei, drei oder vier Substituenten, ausgewählt aus der Gruppe α, aufweisen kann,
und worin
B aus einer der folgenden Gruppen a) oder b) ausgewählt ist, wobei
   a) ein mono-, di- uind trisubstituierter Arylrest ist, wobei der Arylrest Phenyl, Naphthyl oder Phenanthryl ist;
   b) ein un-, mono- und disubstituierter Heteroarylrest ist, wobei der Heteroarylrest Pyridyl, Furanyl, Thienyl, Benzofuranyl, Benzothienyl, 1,2,3,4-Tetrahydrocarbazolyl
und Julolidinyl ist;
wobei die Substituenten der Aryl- und Heteroarylreste in a) und b) solche sind, ausgewählt aus der Gruppe α oder der Gruppe χ, bestehend aus Hydroxy, Amino, Mono-(C₁ - C₆)-alkylamino, Di-(C₁ - C₆)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
oder wobei zwei direkt benachbarte Substituenten der Aryl- und Heteroarylreste in a) und b) eine V-(CR¹²R¹³)ₚ-W-Gruppierung darstellen, wobei p = 1, 2 oder 3 ist, R¹² und R¹³ Wasserstoff, Methyl oder Phenyl sein kann und V und W unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte C-Atome dieser V-(CR¹²R¹³)p-W-Gruppierung jeweils unabhängig voneinander auch Teil eines annellierten Benzo-Ringsystems sein können, welches wiederum einen oder mehrere Substituenten, ausgewählt aus der Gruppe α oder der Gruppe χ, aufweisen kann bzw. können;
und wobei der Arylrest oder Heteroarylrest der Gruppe B in beiden ortho-Stellungen zur Verknüpfungsstelle an den Pyranring keinen Substituenten außer Wasserstoff aufweist,
und worin C ein Substituent der folgenden Struktur ist, 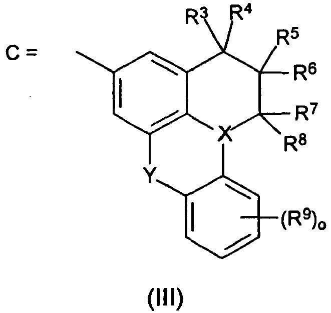
wobei R⁹ wie R¹ und R² definiert ist, wobei m, n und o unabhängig voneinander eine ganze Zahl von 1 bis 4 darstellen;
wobei R³ bis R⁸ unabhängig voneinander jeweils einen Substituenten darstellen, ausgewählt aus der Gruppe α; oder
R³ zusammen mit R⁴ oder R⁵ zusammen mit R⁶ oder R⁷ zusammen mit R⁸ unabhängig voneinander mit dem jeweilig gemeinsamen Kohlenstoffatom einen 3-bis 8-gliedrigen carbo- oder heteromonocyclischen Ring bilden, der gegebenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können; oder
vier benachbarte Reste R³ bis R⁶ oder R⁵ bis R⁸ einen un-, mono- oder disubstituierten, an das Grundgerüst annellierten Benzo- oder Pyridoring bilden, dessen Substituenten aus der Gruppe α ausgewählt sein können;
wobei X für N oder CR¹⁴ steht, wobei R¹⁴ aus der Gruppe, bestehend aus Wasserstoff, einem (C₁-C₆)-Alkylrest und un-, mono- und disubstituiertem Phenyl, dessen Substituenten aus der Gruppe α ausgewählt sind, ausgewählt ist; und
wobei Y für O, S, NR¹⁵, CR¹⁵R¹⁶ oder CR¹⁵R¹⁶-CR¹⁵R¹⁶ steht, wobei R¹⁵ und R¹⁶ jeweils wie R¹⁴ ausgewählt sein können, oder die beiden Reste R¹⁵ und R¹⁶ zusammen mit dem gemeinsamen Kohlenstoffatom einen 3- bis 8-gliedrigen carbo- oder heteromonocyclischen Ring bilden, der gegebenenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können.

Die erfindungsgemäßen photochromen annellierten Naphthopyrane sind durch einen zum Pyransauerstoff benachbarten Benzolring, der über beide meta-Stellungen mit dem para-Substituenten verknüpft ist, gekennzeichnet. Die erfindungsgemäßen Verbindungen zeichnen sich durch sehr hohe Eindunklungsleistungen auch bei höheren Temperaturen, schnelle Aufhellgeschwindigkeiten sowie sehr gute Lichtbeständigkeiten aus. Bevorzugt weisen die erfindungsgemäßen photochromen annellierten Naphthopyrane die vorstehende allgemeine Formel (I) auf.

Besonders bevorzugte photochrome annellierte Naphthopyrane gemäß der vorliegenden Erfindung mit einem zum Pyransauerstoff benachbarten Benzolring, der über beide meta-Stellungen mit dem para-Substituenten verknüpft ist, weisen die nachfolgenden allgemeinen Formeln (III ), (IV) und (V) auf: 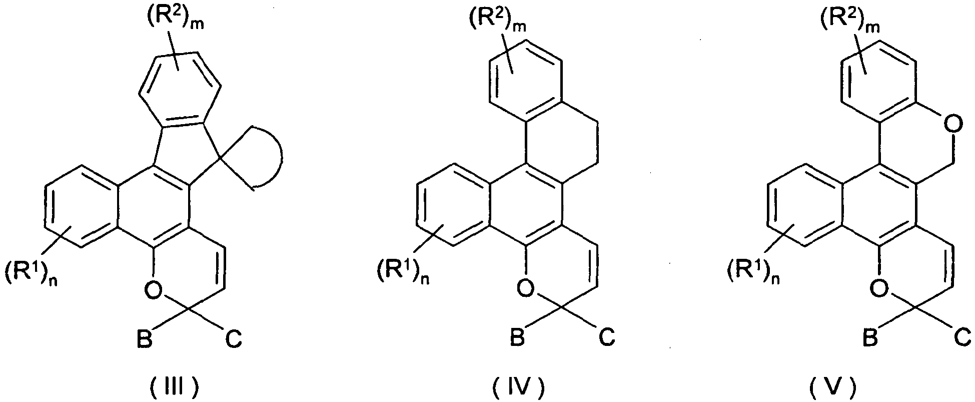 worin sämtliche Reste wie vorstehend definiert sind und worin der Spiro-Ring in Formel (III) unter Einbeziehen des Spiro-Kohlenstoffatoms einen 5- bis 8-gliedrigen carbocyclischen Ring darstellt, der gegebenfalls einen oder mehrere Substituenten aus der Gruppe α trägt, und an den ein bis drei Benzolringe annelliert sein können, die wiederum mit einem oder mehreren Substituenten, ausgewählt aus der Gruppe α, substituiert sein können.

In einer bevorzugten Ausführungsform der vorliegenden Erfindung ist der Rest B aus der Gruppe a) ausgewählt. In einer weiteren Ausführungsform ist X im Rest C vorzugsweise N. Y steht im Rest C vorzugsweise für CR¹⁵R¹⁶ oder CR¹⁵R¹⁶-CR¹⁵R¹⁶, wobei R¹⁵ bzw. R¹⁶ wie vorstehend definiert sind und vorzugsweise aus der Gruppe sind, bestehend aus Wasserstoff, einem (C₁ - C₆)-Alkylrest und un-, mono- und disubstituiertem Phenyl, dessen Substituenten aus der Gruppe α ausgewählt sind. Die Reste R³ bis R⁸ stellen vorzugsweise unabhängig voneinander jeweils einen Substituenten, ausgewählt aus der Gruppe α, dar.

In einer Ausführungsform der vorliegenden Erfindung stellen die Reste R³ und R⁴ unabhängig voneinander jeweils einen Substituenten, ausgewählt aus der Gruppe α, und die vier Reste R⁵ bis R⁸ gemeinsam einen un-, mono- oder disubstituierten, an das Grundgerüst annellierten Benzo- oder Pyridoring, dessen Substituenten aus der Gruppe α ausgewählt sein können, dar.

In einer besonders bevorzugten Ausführungsform weist der Rest C folgende Strukturen auf:

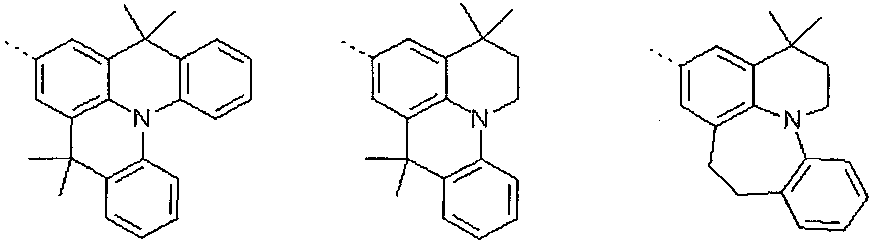

Des weiteren bilden in einer weiteren Ausführungsform zwei Reste R² in den vorstehenden Formeln (I)-(V) einen un-, mono- oder disubstituierten, an das Grundgerüst annellierten Benzo-, Pyrido-, Benzofuro- oder Benzothienoring, dessen Substituenten aus der Gruppe α ausgewählt sein können. Besonders bevorzugt stehen zwei in ortho-Position zueinander vorliegende Reste R² in den vorstehenden Formeln (I)-(V) für eine Benzofuro-Annellierung.

Z ist vorzugsweise aus -CR¹⁰R¹¹-, -O-CR¹⁰R¹¹-, -CR¹⁰R¹¹-O-, -CR¹⁰R¹¹-CR¹⁰R¹¹ oder -CR¹⁰=CR¹¹- ausgewählt, wobei die Substituenten R¹⁰ und R¹¹ aus der Gruppe α, vorzugsweise aus einem Wasserstoffatom, einem (C₁ - C₆)-Alkylrest, einem (C₃ - C₇)-Cycloalkylrest, einem un-, mono- oder disubstituiertem Phenyl-, Benzyl- oder Naphthylrest, ausgewählt sind.

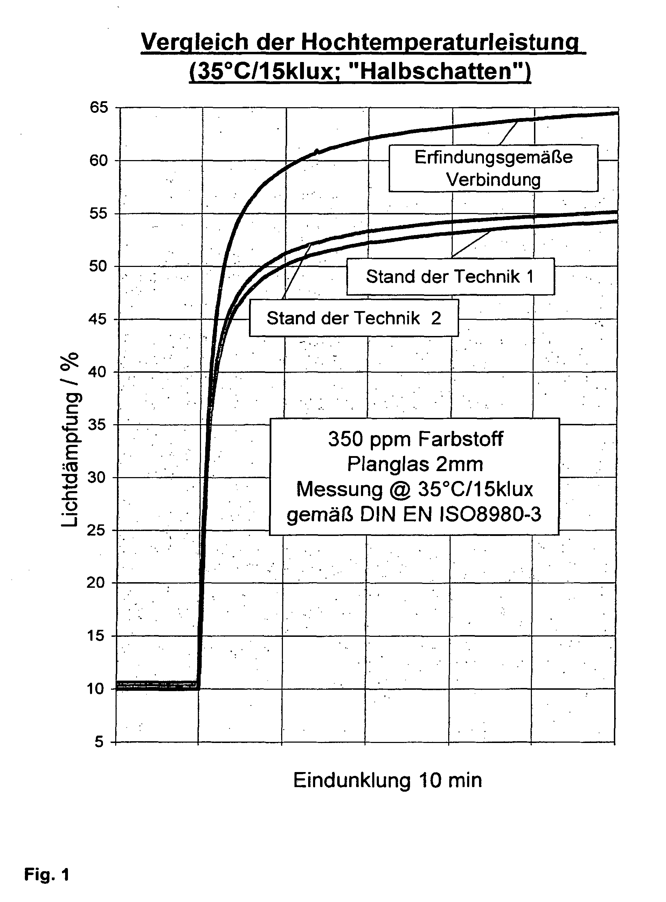

Die Figur 1 zeigt einen Vergleich der Lichtdämpfungseigenschaften im angeregten Zustand bei höherer Temperatur und schwächerem Anregungslicht ("Hochtemperatirrleistung Halbschatten") einer erfindungsgemäßen Verbindung im Vergleich zu Verbindungen aus dem Stand der Technik.

Ein Problem herkömmlicher phototroper Farbstoffe ist eine oft mangelhafte Hochtemperaturleistung, da bei höheren Temperaturen die (thermische) Rückreaktion der angeregten (farbigen) Form so schnell erfolgt, dass die Eindunklungstiefe relativ gering ausfällt. Es wird jedoch von modernen phototropen Gläsern auch eine hinreichend tiefe Eindunklung bei höheren Temperaturen verlangt, und zwar dies auch im Halbschatten mit relativ wenig UV-Anregung. Daher ist die Kinetikbank-Messung bei 35°C und mit nur 15klux Anregungslicht - statt dem Standardwert von 50klux für volles Sonnenlicht - ein gutes Mass für die Hochtemperaturleistung phototroper Farbstoffe. Wie in Figur 1 dargestellt, weisen erfindungsgemäße Verbindungen mit dem spezifisch gewählten Substituenten C eine etwa 10% höhere Lichtdämpfung bei 35°C/15klux- d.h. eine 10% niedrigere Transmission - im Vergleich zu Verbindungen aus dem Stand der Technik mit analoger Naphthopyran-Untereinheit und gleicher Farbstoffkonzentration auf.

Bisher war bekannt, dass die Eindunklungstiefe eines photochromen Farbstoffes im angeregten Zustand erhöht werden kann, wenn am Benzolring benachbart zum Pyran-Sauerstoff kleine ortho-Substituenten wie Fluor, Methyl oder Methoxy vorgesehen werden. Diese Verbindungen dunkeln dann bei Anregung zwar extrem stark ein, jedoch wird dadurch die Aufhellgeschwindigkeit so stark verlangsamt, dass sie für den beabsichtigten Verwendungszweck in Brillengläsern nicht verwendbar sind. Im Gegensatz dazu hellen die erfindungsgemäßen Verbindungen, die am Benzolring benachbart zum Pyran-Sauerstoff zwei meta-Substituenten aufweisen, nach vollständiger Eindunklung wieder schnell auf. Der Grund dafür liegt in der besonderen Struktur des Substituenten C mit mindestens vier annellierten Ringen.

Da die erfindungsgemäßen Verbindungen darüber hinaus hohe Augenklarheit (d.h. hohe Transmission im nicht angeregten Zustand) sowie sehr gute Lichtbeständigkeit aufweisen, sind sie zum Einsatz in phototropen Gläsern speziell für wärmere Klimazonen oder als Zumischung für konventionelle phototrope Gläser zur Erhöhung der Eindunklungstiefe bei höheren Temperaturen geeignet.

Die Struktur der in Figur 1 eingesetzten Verbindungen des Standes der Technik (aus WO2009/024271) sowie einer erfindungsgemäßen Verbindung sind aus der folgenden Tabelle ersichtlich:

**Tabelle 1: Struktur einer erfindungsgemäßen Verbindung vs. Stand der Technik**

| | |
|---|---|
| 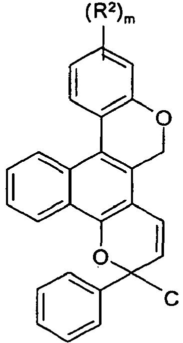 | 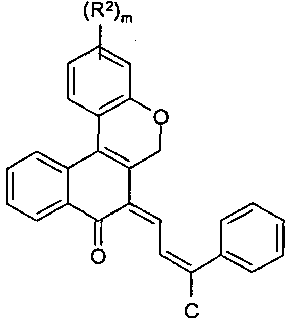 |
| nicht angeregt (farblos) | angeregt (farbig) |
| Substituent (R²)ₘ = Benzofuro-Annellierung (gemäß WO 2009/024271) | |
| Substituent C ist jeweils nachstehend abgebildet | |

Nachfolgend sind die Substituenten C der Verbindungen 1 bzw. 2 gemäß dem Stand der Technik (aus WO2009/024271) sowie einer erfindungsgemäßen Verbindung angeführt:

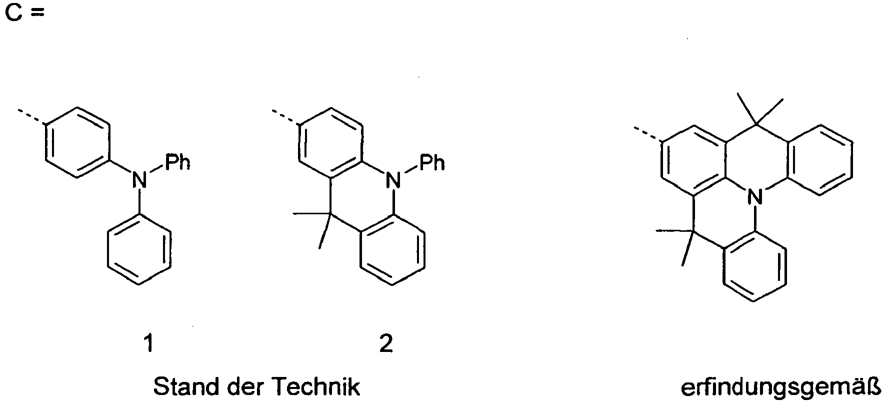

Zur Messung der Eigenschaften des vorstehend aufgeführten, beispielhaften erfindungsgemäßen photochromen Farbstoffes sowie der Verbindungen aus dem Stand der Technik (s.o.) wurden jeweils 350 ppm des Farbstoffes in einer Acrylatmonomer-Matrix gelöst und nach Zusatz eines Polymerisations-Initiators mit Hilfe eines Temperaturprogrammes thermisch polymerisiert. Die Lichtdämpfungs- bzw. Transmissionseigenschaften der so hergestellten Kunststoffgläser (Dicke 2 mm) wurden anschließend nach DIN EN ISO 8980-3 vermessen.

In Tabelle 2 ist ein Intensitätsvergleich der Lichtdämpfung im angeregten Zustand (gemessen direkt beim jeweiligen längstwelligen Absorptionsmaximum) von zwei weiteren erfindungsgemäßen Verbindungen mit dem Stand der Technik aufgeführt. In der Formel ist jeweils nur der Substituent C abgebildet - das restliche Molekül ist analog zu den Verbindungen aus Tabelle 1 aufgebaut.

**Tabelle 2: Intensitätsvergleich der Lichtdämpfung im angeregten Zustand (35°C/15klux); Messung voll eingedunkelt direkt beim längstwelligen Absorptionsmaximum (vollständige Formel siehe Tabelle 1; nur Substituent C abgebildet)**

| | | |
|---|---|---|
| Stand der Technik | Erfindungsgemäße | Verbindungen |
| 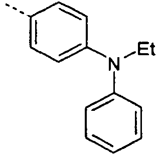 | 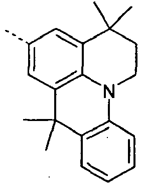 | 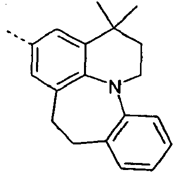 |
| Lichtdämpfung @35°C/15klux | | |
| 58% | 70% | 68% |
| längstwelliges Absorptionsmax. | | |
| 590nm | 610nm | 590nm |

Die erfindungsgemäßen Verbindungen weisen jeweils eine etwa 10% höhere Lichtdämpfung im angeregten Zustand bei höheren Temperaturen auf als vergleichbare Verbindungen aus dem Stand der Technik.

Die Herstellung von Verbindungen der Formel (V), die in den Tabellen 1 und 2 aufgeführt sind, erfolgt analog WO 2009/024271, wobei im letzten Schritt die Kondensation mit 2-Propin-1-ol-derivaten erfolgt, die den erfindungsgemäß gewählten Substituenten C anstelle von B' enthalten. Diese lassen sich nach den üblichen Verfahren aus den entsprechend substituierten Benzophenonen darstellen. Verbindungen der Formel (IV) lassen sich analog in Anlehnung an WO2009/132842 synthetisieren, die Verbindungen der Formel (III) in Anlehnung an EP 0 987 260.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Naphthopyrane in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Naphthopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen.

Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome Naphthopyrane gemäß nachstehender Formel (I) oder (II): 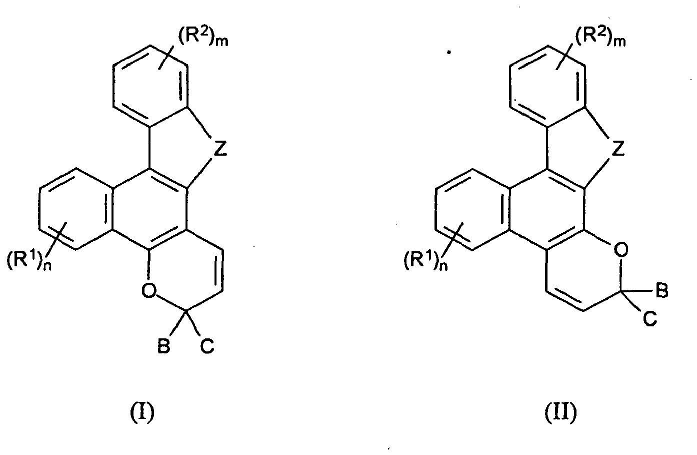 worin
die Reste R¹ und R² jeweils unabhängig voneinander einen Substituenten darstellen, ausgewählt aus der Gruppe α, bestehend aus einem Wasserstoffatom, einem (C₁ - C₆)-Alkylrest, einem (C₁ - C₆)-Thioalkylrest, einem (C₃ - C₇)-Cycloalkylrest, der ein oder mehrere Heteroatome, wie beispielsweise O oder S, aufweisen kann, einem(C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder
zwei Reste R¹ und/oder zwei Reste R² jeweils unabhängig voneinander einen un-, mono- oder disubstituierten, an das Grundgerüst annellierten Benzo-, Pyrido-, Benzofuro- oder Benzothienoring bilden, dessen Substituenten aus der Gruppe α ausgewählt sein können;
Z aus -CR¹⁰R¹¹-, -O-, -S-, -NPh-, -N(C₁-C₆)Alkyl, -O-CR¹⁰R¹¹-, -CR¹⁰R¹¹-O-, -S-CR¹⁰R¹¹-, -CR¹⁰R¹¹-S-, -CR¹⁰R¹¹-CR¹⁰R¹¹-, -CR¹⁰=N- oder -CR¹⁰=CR¹¹- ausgewählt ist, wobei die Substituenten R¹⁰ und R¹¹ aus der Gruppe α ausgewählt sein können;
oder Z unter Einbeziehen des Spiro-Kohlenstoffatoms einen 3- bis 8-gliedrigen carbo- oder heteromonozyklischen Ring darstellt, der gegebenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan; Thiophen, Pyrrol, Benzofuran, Benzothiophen, Indol und Carbazol, die wiederum mit einem oder mehreren Substituenten, ausgewählt aus der Gruppe α, substituiert sein können; oder Z unter Einbeziehen des Spiro-Kohlenstoffatoms einen 7- bis 12-gliedrigen carbo-bizyklischen Ring bzw. einen 7- bis 12-gliedrigen carbo-trizyklischen Ring darstellt, der wiederum einen, zwei, drei oder vier Substituenten, ausgewählt aus der Gruppe α, aufweisen kann,
und worin
B aus einer der folgenden Gruppen a) oder b) ausgewählt ist, wobei
a) ein mono-, di- uind trisubstituierter Arylrest ist, wobei der Arylrest Phenyl, Naphthyl oder Phenanthryl ist;
b) ein un-, mono- und disubstituierter Heteroarylrest ist, wobei der Heteroarylrest Pyridyl, Furanyl, Thienyl, Benzofuranyl, Benzothienyl, 1,2,3,4-Tetrahydrocarbazolyl und Julolidinyl ist;
wobei die Substituenten der Aryl- und Heteroarylreste in a) und b) solche sind, ausgewählt aus der Gruppe α oder der Gruppe χ, bestehend aus Hydroxy, Amino, Mono-(C₁ - C₆)-alkylamino, Di-(C₁ - C₆)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl un-, mono- oder disubsti-tuiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
oder wobei zwei direkt benachbarte Substituenten der Aryl- und Heteroarylreste in a) und b) eine V-(CR¹²R¹³)ₚ-W-Gruppierung darstellen, wobei p = 1, 2 oder 3 ist, R¹² und R¹³ Wasserstoff, Methyl oder Phenyl sein kann und V und W unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte C-Atome dieser V-(CR¹²R¹³)ₚ-W-Gruppierung jeweils unabhängig voneinander auch Teil eines annellierten Benzo-Ringsystems sein können, welches wiederum einen oder mehrere Substituenten, ausgewählt aus der Gruppe α oder der Gruppe χ, aufweisen kann bzw. können;
und wobei der Arylrest oder Heteroarylrest der Gruppe B in beiden ortho-Stellungen zur Verknüpfungsstelle an den Pyranring keinen Substituenten außer Wasserstoff aufweist,
und worin C ein Substituent der folgenden Struktur ist, 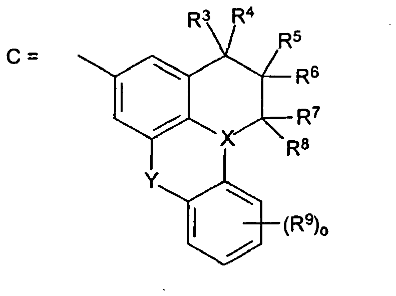
wobei R⁹ wie R¹ und R² definiert ist, wobei m, n und o unabhängig voneinander eine ganze Zahl von 1 bis 4 darstellen;
wobei R³ bis R⁸ unabhängig voneinander jeweils einen Substituenten darstellen, ausgewählt aus der Gruppe α; oder
R³ zusammen mit R⁴ oder R⁵ zusammen mit R⁶ oder R⁷ zusammen mit R⁸ unabhängig voneinander mit dem jeweilig gemeinsamen Kohlenstoffatom einen 3-bis 8-gliedrigen carbo- oder heteromonocyclischen Ring bilden, der gegebenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können; oder
vier benachbarte Reste R³ bis R⁶ oder R⁵ bis R⁸ einen un-, mono- oder disubstituierten, an das Grundgerüst annellierten Benzo- oder Pyridoring bilden, dessen Substituenten aus der Gruppe α ausgewählt sein können;
wobei X für N oder CR¹⁴ steht, wobei R¹⁴ aus der Gruppe, bestehend aus Wasserstoff, einem (C₁-C₆)-Alkylrest und un-, mono- und disubstituiertem Phenyl, dessen Substituenten aus der Gruppe α ausgewählt sind, ausgewählt ist; und wobei Y für O, S, NR¹⁵, CR¹⁵R¹⁶ oder CR¹⁵R¹⁶-CR¹⁵R¹⁶ steht, wobei R¹⁵ und R¹⁶ jeweils wie R¹⁴ ausgewählt sein können, oder die beiden Reste R¹⁵ und R¹⁶ zusammen mit dem gemeinsamen Kohlenstoffatom einen 3- bis 8-gliedrigen carbo- oder heteromonocyclischen Ring bilden, der gegebenenfalls einen oder mehrere Substituenten aus der Gruppe α trägt, und an den ein bis drei aromatische oder heteroaromatische Ringsysteme annelliert sein können, wobei das bzw. die Ringsysteme unabhängig voneinander aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können.

2. Photochrome Naphthopyrane gemäß Anspruch 1, welche die Formel (I) aufweisen.

3. Photochrome Naphthopyrane gemäß Anspruch 1 oder 2, welche die nachfolgenden allgemeinen Formeln ( III ), ( IV ) und ( V ) aufweisen: 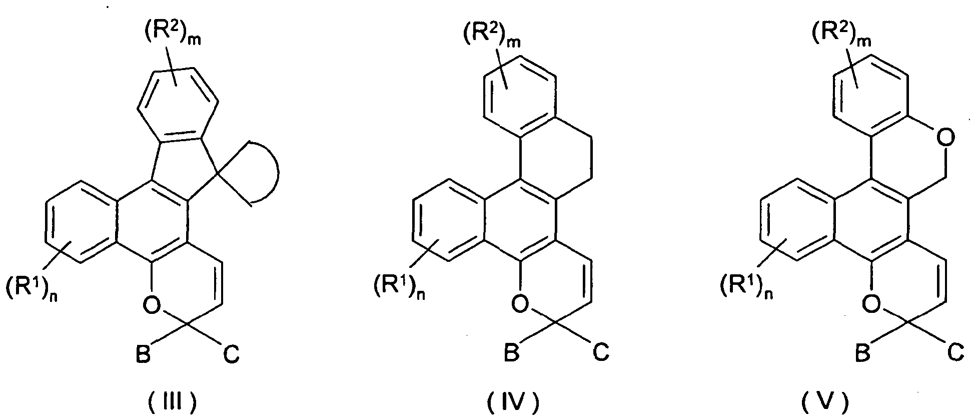 worin sämtliche Reste wie vorstehend definiert sind und worin der Spiro-Ring in Formel (III) unter Einbeziehen des Spiro-Kohlenstoffatoms einen 5- bis 8-gliedrigen carbocyclischen Ring darstellt, der gegebenfalls einen oder mehrere Substituenten aus der Gruppe α trägt, und an den ein bis drei Benzolringe annelliert sein können, die wiederum mit einem oder mehreren Substituenten, ausgewählt aus der Gruppe α, substituiert sein können.

4. Photochrome Naphthopyrane gemäß einem der Ansprüche 1 bis 3, wobei der Rest B aus der Gruppe a) ausgewählt ist.

5. Photochrome Naphthopyrane gemäß einem der Ansprüche 1 bis 4, wobei X im Rest C für N steht.

6. Photochrome Naphthopyrane gemäß einem der Ansprüche 1 bis 5, wobei Y im Rest C für CR¹⁵R¹⁶ oder CR¹⁵R¹⁶-CR¹⁵R¹⁶ steht, wobei R¹⁵ bzw. R¹⁶ wie vorstehend definiert sind.

7. Photochrome Naphthopyrane gemäß einem der Ansprüche 1 bis 6, wobei R³ bis R⁸ unabhängig voneinander jeweils einen Substituenten darstellen, ausgewählt aus der Gruppe α.

8. Photochrome Naphthopyrane gemäß einem der Ansprüche 1 bis 7, wobei wobei R³ und R⁴ unabhängig voneinander jeweils einen Substituenten darstellen, ausgewählt aus der Gruppe α; und gleichzeitig die vier Reste R⁵ bis R⁸ gemeinsam einen un-, mono- oder disubstituierten, an das Grundgerüst annellierten Benzo- oder Pyridoring bilden, dessen Substituenten aus der Gruppe α ausgewählt sein . können.

9. Photochrome Naphthopyrane gemäß einem der Ansprüche 1 bis 8, wobei der Rest C folgende Strukturen aufweist.

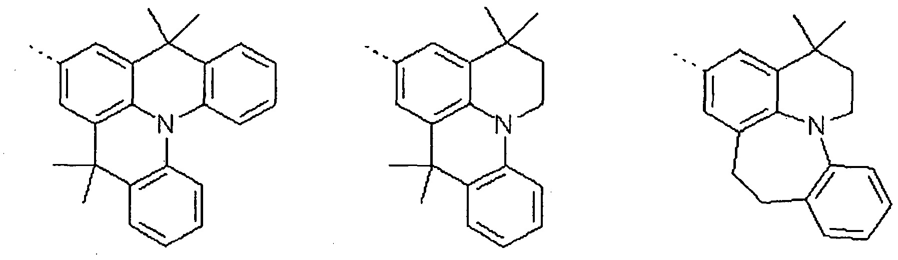

10. Photochrome Naphthopyrane gemäß einem der Ansprüche 1 bis 9, wobei zwei Reste R² in den vorstehenden Formeln (I)-(V) einen un-, mono- oder disubstituierten, an das Grundgerüst annellierten Benzo-, Pyrido-, Benzofuro- oder Benzothienoring bilden, dessen Substituenten aus der Gruppe α ausgewählt sein können;

11. Photochrome Naphthopyrane gemäß einem der Ansprüche 1 bis 10, wobei zwei in ortho-Position zueinander vorliegende Reste R² in den vorstehenden Formeln (I)-(V) für eine Benzofuro-Annellierung stehen.

12. Photochrome Naphthopyrane gemäß einem der Ansprüche 1 bis 11, wobei Z aus -CR¹⁰R¹¹-, -O-CR¹⁰R¹¹-, -CR¹⁰R¹¹-O-, -CR¹⁰R¹¹-CR¹⁰R¹¹ oder -CR¹⁰=CR¹¹-ausgewählt ist, wobei die Substituenten R¹⁰ und R¹¹ aus der Gruppe α ausgewählt sind.

13. Verwendung der photochromen Naphthopyrane nach einem der Ansprüche 1 bis 12 in und auf Kunststoffmaterialien.

14. Verwendung nach Anspruch 13, wobei das Kunststoffmaterial eine ophthalmische Linse ist.

## Claims

1. Photochromic naphthopyrans according to the following formula (I) or (II): 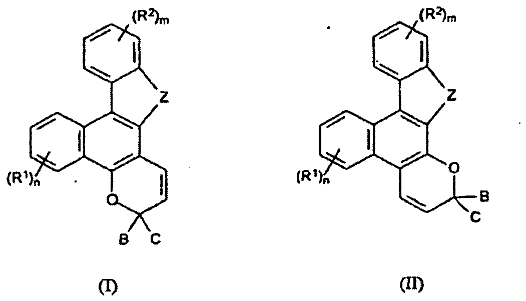
wherein radicals R¹ and R², independently of one another, respectively represent a substituent selected from the α group consisting of a hydrogen atom, a (C₁-C₆) alkyl radical, a (C₁-C₆) thioalkyl radical, a (C₃-C₇) cycloalkyl radical, which can have one or more heteroatoms such as O or S, for example, a (C₁-C₆) alkoxy radical, a hydroxy group, a trifluoromethyl group, bromine, chlorine, fluorine, an un-, mono- or disubstituted phenyl, phenoxy, benzyl, benzyloxy, naphthyl or naphthoxy radical, wherein the substituents can themselves be selected from the α group; or
two R¹ radicals and/or two R² radicals, independently of one another, respectively form an un-, mono- or disubstituted benzo, pyrido, benzofuro or benzothieno ring annellated to the base structure, the substituents of which ring can be selected from the α group;
Z is selected from -CR¹⁰R¹¹- -O-, -S-, -NPh-, -N(C₁-C₆)alkyl, -O-CR¹⁰R¹¹-, -CR¹⁰R¹¹-O-, -S-CR¹⁰⁻R¹¹-, -CR¹⁰R¹¹-S-, -CR¹⁰R¹¹-CR¹⁰R¹¹-, -CR¹⁰=10=N- or -CR¹⁰=CR¹¹, wherein substituents R¹⁰ and R¹¹ can be selected from the α group;
or Z with incorporation of the spiro carbon atom represents a 3- to 8-membered carbo- or heteromonocyclic ring, which ring possibly bears one or more substituents from the α group, and onto which one to three aromatic or heteroaromatic ring systems can be annellated, wherein the ring system or systems are selected independently from one another from the β group consisting of benzene, naphthalene, phenanthrene, pyridine, quinoline, furan, thiophene, pyrrole, benzofuran, benzothiophene, indole and carbazole, which can themselves be substituted with one or more substituents selected from the α group;
or Z with incorporation of the spiro carbon atom represents a 7- to 12-membered carbo-bicylic ring or a 7- to 12-membered carbo-tricyclic ring, which ring can itself have one, two, three or four substituents selected from the α group,
and wherein
B is selected from the following groups a) or b), wherein
a) is a mono-, di- and trisubstituted aryl radical, wherein the aryl radical is phenyl, naphthyl or phenanthryl;
b) is an un-, mono- and disubstituted heteroaryl radical, wherein the heteroaryl radical is pyridyl, furanyl, thienyl, benzofuranyl, benzothienyl, 1,2,3,4-tetrahydrocarbazolyl and julolidinyl;
wherein the substituents of the aryl and heteroaryl radicals in a) and b) are those selected from the α group or the χ group consisting of hydroxy, amino, mono-(C₁-C₆) alkylamino, di-(C₁-C₆) alkylamino, phenethenyl un-, mono- or disubstituted on the phenyl ring, un-, mono- or disubstituted (phenyl-imino)methylene, un-, mono- or disubstituted (phenylmethylene)imino and un-, mono- or disubstituted mono- and diphenylamino, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted 1,2,3,4-tetrahydrocarbazolyl and un-, mono- or disubstituted 10,11-dihydrodibenz[b,f]azepinyl, wherein the substituent or substituents, independently of one another, can themselves be selected from (C₁-C₆) alkyl, (C₁-C₆) alkoxy, bromine, chlorine or fluorine;
or wherein two directly adjacent substituents of the aryl and heteroaryl radicals in a) and b) represent a V-(CR¹²R¹³)ₚ W-group, wherein p = 1, 2 or 3, R¹² and R¹³ can be hydrogen, methyl or phenyl and V and W, independently of one another, can be oxygen, sulphur, N-(C₁-C₆) alkyl, N-C₆H₅, CH₂, C(CH₃)₂ or C(C₆H₅)₂, wherein two or more adjacent C atoms of this V-(CR¹²R¹³)ₚ W-group, independently of one another, can also themselves be part of an annellated benzo ring system, which can in turn have one or more substituents selected from the α group or the χ group;
and wherein in both ortho positions to the linkage point to the pyran ring the aryl radical or heteroaryl radical of group B has no substituents except hydrogen, and wherein C is a substituent of the following structure 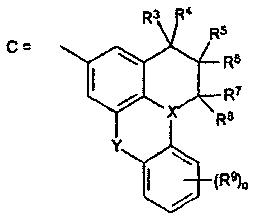
wherein R⁹ is defined like R¹ and R², wherein m, n and o, independently of one another, represent a whole number from 1 to 4;
wherein R³ to R⁸, independently of one another, respectively represent a substituent selected from the α group; or
R³ together with R⁴ or R⁵ together with R⁶ or R⁷ together with R⁸, independently of one another, form with the respectively common carbon atom a 3- to 8-membered carbo- or heteromonocyclic ring, which possibly bears one or more substituents from the α group, and can be annellated onto the one to three aromatic or heteroaromatic ring systems, wherein the ring system or systems are selected independently of one another from the β group consisting of benzene, naphthalene, phenanthrene, pyridine, quinoline, furan, thiophene, pyrrole, benzofuran, benzothiophene, indole and carbazole, which can themselves be substituted with one or more substituents selected from the α group; or
four adjacent R³ to R⁶ or R⁵ to R⁸ radicals form an un-, mono- or disubstituted benzo or pyrido ring annellated onto the base structure, the substituents of which ring can be selected from the α group;
wherein X stands for N or CR¹⁴, wherein R¹⁴ is selected from the group consisting of hydrogen, a (C₁-C₆) alkyl radical and un-, mono- and disubstituted phenyl, the substituents of which are selected from the α group; and
wherein Y stands for O, S, NR¹⁵, CR¹⁵R¹⁶ or CR¹⁵R¹⁶-CR¹⁵R¹⁶, wherein R¹⁵ and R¹⁶ can be respectively selected like R¹⁴ or the two radicals R¹⁵ and R¹⁶ together with the common carbon atom form a 3- to 8-membered carbo- or heteromonocyclic ring, which possibly bears one or more substituents from the α group, and onto which one to three aromatic or heteroaromatic ring systems can be annellated, wherein the ring system or systems are selected independently of one another from the β group consisting of benzene, naphthalene, phenanthrene, pyridine, quinoline, furan, thiophene, pyrrole, benzofuran, benzothiophene, indole and carbazole, which can themselves be substituted with one or more substituents selected from the α group.

2. Photochromic naphthopyrans according to claim 1, which have the formula (I).

3. Photochromic naphthopyrans according to claim 1 or 2, which have the following general formulae (III), (IV) and (V): 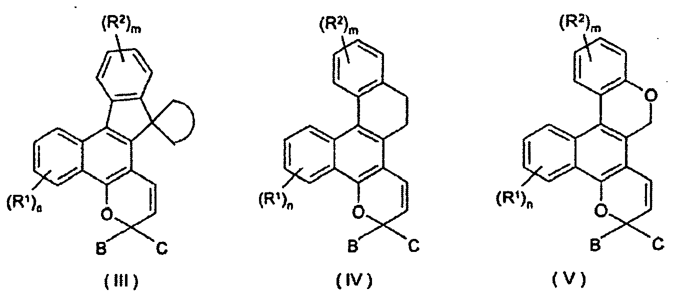 wherein all the radicals are defined as above and wherein with incorporation of the spiro carbon atom the spiro ring in formula (III) represents a 5- to 8-membered carbocyclic ring, which possibly bears one or more substituents from the α group, and onto which one to three benzene rings can be annellated, which can themselves be substituted with one or more substituents selected from the α group.

4. Photochromic naphthopyrans according to one of claims 1 to 3, wherein radical B is selected from group a).

5. Photochromic naphthopyrans according to one of claims 1 to 4, wherein X in radical C stands for N.

6. Photochromic naphthopyrans according to one of claims 1 to 5, wherein Y in radical C stands for CR¹⁵R¹⁶ or CR¹⁵R¹⁶-CR¹⁵R¹⁶, wherein R¹⁵ and R¹⁶ are respectively defined as above.

7. Photochromic naphthopyrans according to one of claims 1 to 6, wherein R³ to R⁸, independently of one another, respectively represent a substituent selected from the a group.

8. Photochromic naphthopyrans according to one of claims 1 to 7, wherein R³ and R⁴, independently of one another, respectively represent a substituent selected from the α group; and at the same time the four radicals R⁵ to R⁸ together form an un-, mono- or disubstituted benzo or pyrido ring annellated onto the base structure, the substituents of which ring can be selected from the α group.

9. Photochromic naphthopyrans according to one of claims 1 to 8, wherein radical C has the following structures:

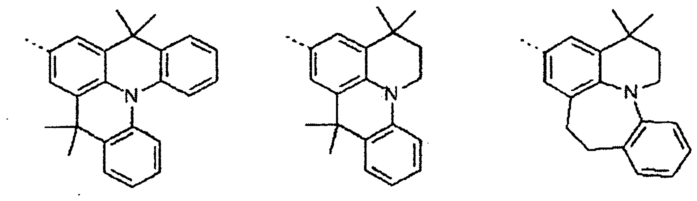

10. Photochromic naphthopyrans according to one of claims 1 to 9, wherein two R² radicals in the above formulae (I)-(V) form an un-, mono- or disubstituted benzo, pyrido, benzofurano or benzothieno ring annellated onto the base structure, the substituents of which ring can be selected from the α group.

11. Photochromic naphthopyrans according to one of claims 1 to 10, wherein two R² radicals present in ortho position relative to one another in the above formulae (I)-(V) stand for a benzofuro annellation.

12. Photochromic naphthopyrans according to one of claims 1 to 11, wherein Z is selected from -CR¹⁰R¹¹- O-CR¹⁰R¹¹-, -CR¹⁰R¹¹-O-, CR¹⁰-R¹¹-CR¹⁰R¹¹ or -CR¹⁰=CR¹¹, wherein substituents R¹⁰ and R¹¹ are selected from the α group.

13. Use of the photochromic naphthopyrans according to one of claims 1 to 12 in and on plastic materials.

14. Use according to claim 13, wherein the plastic material is an ophthalmic lens.

## Revendications

1. Naphtopyranes photochromes selon les formules (I) ou (II) suivantes : 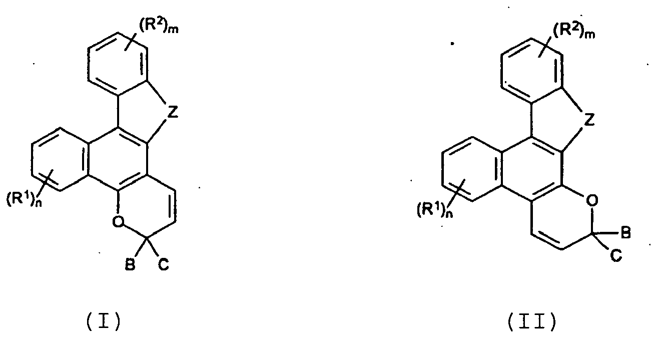
dans lesquelles
les radicaux R¹ et R² représentent indépendamment les uns des autres un substituant choisi dans le groupe α constitué par un atome d'hydrogène, un groupe alkyle en C₁-C₆, un groupe thioalkyle en C₁-C₆, un groupe cycloalkyle en C₃-C₇, qui peut présenter un ou plusieurs hétéroatomes, comme, par exemple, O ou S, un groupe alcoxy en C₁-C₆, un groupe hydroxyle, un groupe thiofluorométhyle, du brome, du chlore, du fluor, des groupes phényle, phénoxy, benzyle, benzyloxy, naphtyle ou naphtoxy non substitués, monosubstitués ou disubstitués, les substituants pouvant, à leur tour, être choisis dans le groupe α ; ou deux radicaux R¹ et/ou deux radicaux R² peuvent former, indépendamment les uns des autres, un cycle, cyclisé sur le squelette de base benzénique, pyridynique, benzofuranique ou benzothionique, non substitué, mono- ou disubstitué, dont les substituants peuvent être choisis dans le groupe α ;
Z peut être choisi dans les groupes -CR¹⁰R¹¹⁻, -O-, -S-, -NPh-, -N-alkyle en C₁-C₆, -O-CR¹⁰R¹¹-, -CR¹⁰R¹⁰-O-, -S-CR¹⁰R¹¹, -CR¹⁰R¹¹-S-, -CR¹⁰R¹¹CR¹⁰R¹¹, -CR¹⁰=N- ou - CR¹⁰=CR¹¹, les substituants R¹⁰ et R¹¹ pouvant être choisis dans le groupe α
ou Z représente un cycle monocyclique, carbo- ou hétérocyclique, de 3 à 8 chaînons, moyennant l'incorporation de l'atome de carbone spiro, qui porte éventuellement un ou plusieurs substituants faisant partie du groupe α**,** et sur lequel peuvent être cyclisés de un à trois systèmes cycliques aromatiques ou hétéro-aromatiques, les systèmes cycliques étant par exemple choisis indépendamment les uns des autres dans le groupe β, constitué par le benzène, le naphtalène, le phénanthrène, la pyridine, la quinoléine, le furane, le thiophène, le pyrrole, le benzofurane, le benzothiophène, l'indole et le carbazole, qui, à leur tour, peuvent être substitués avec un ou plusieurs substituants choisis dans le groupe α ;
ou Z représente un cycle carbo-bicyclique de 7 à 12 chaînons, respectivement, un cycle carbo-tricyclique de 7 à 12 chaînons, moyennant l'incorporation de l'atome de carbone spiro, qui, à son tour, peut présenter un, deux, trois ou quatre substituants choisis dans le groupe α,
et dans lesquelles
B est un groupe choisi parmi les groupes a) ou b) suivants,
a) étant un groupe aryle mono-, di- et trisubstitué, le groupe aryle étant un groupe phényle, naphtyle ou phénanthryle ;
b) étant un groupe hétéroaryle non substitué, mono- et disubstitué, le groupe hétéroaryle étant un groupe pyridyle, furanyle, thiényle, benzofuranyle, 1,2,3,4-tétrahydrocarbazolyle et julolidinyle ;
les substituants des groupes aryles et hétéroaryles dans les groupes a) et b) étant ceux choisis dans le groupe α ou dans le groupe χ, constitué par un groupe hydroxyle, amine, mono-alkylamine en C₁-C₆, di-alkylamine en C₁-C₆, sur le cycle phényle, un groupe phénéthényle non substitué, mono- ou disubstitué, un groupe (phényl-imino)méthylène non substitué, mono ou disubstitué, et un groupe mono- et diphénylamine non substitué, mono- ou disubstitué, un groupe pipéridinyle, un groupe pipérazinyle N substitué, un groupe pyrrolidinyle, un groupe imidazolidinyle, un groupe pyrazolodinyle, un groupe indolinyle, un groupe morpholinyle, un groupe 2,6-diméthylmorpholinyle, un groupe thiomorpholinyle, un groupe azacycloheptyle, un groupe azacyclo-octyle, un groupe phénothiazinyle non substitué, mono- ou disubstitué, un groupe phénoxazinyle non substitué, mono- ou disubstitué, un groupe 1,2,3,4-tétrahydroquinoléinyle non substitué, mono- ou disubstitué, un groupe 2,3-dihydro-1,4-benzoxazinyle non substitué, mono- ou disubstitué, un groupe 1,2,3,4-tétrahydroisoquinoléinyle non substitué, mono- ou disubstitué, un groupe phénazinyle non substitué, mono- ou disubstitué, un groupe carbazolyle non substitué, mono- ou disubstitué, un groupe 1,2,3,4-tétrahydrocarbazolyle non substitué, mono- ou disubstitué et un groupe 10,11-dihydrodibenz[b,f]azépinyle non substitué, mono- ou disubstitué, le ou les substituants, à leur tour, pouvant être choisis indépendamment les uns des autres parmi un groupe alkyle en C₁-C₆, un groupe alcoxy en C₁-C₆, du brome, du chlore ou du fluor ;
ou deux substituants directement voisins des groupes aryles et hétéroaryles représentant un groupement V- (CR¹²R¹³)ₚ-W dans les groupes a) et b), p étant égal à 1, 2 ou 3, R¹² et R¹³ pouvant être de l'hydrogène, un groupe méthyle ou phényle, et V et W pouvant être indépendamment l'un de l'autre, de l'oxygène, du soufre, un groupe N-alkyle en C₁-C₆, un groupe N-C₆H₅, un groupe CH₂, un groupe C(CH₃)₂ ou un groupe C(C₆H₅)₂, deux ou trois atomes de C de ce groupement C(CR¹²R¹³)ₚ-W pouvant être, respectivement, indépendamment les uns des autres, également une(des) partie(s) d'un système cyclique benzénique qui, à son(leur) tour, peut présenter, respectivement, peuvent présenter, un ou plusieurs substituants choisis dans le groupe α ou dans le groupe χ ;
et le groupe aryle ou le groupe hétéro aryle du groupe B ne présentent aucun substituant, mis à part l'hydrogène, dans les deux positions ortho pour la liaison sur le cycle pyranne,
et dans lesquelles C est un substituant de la structure suivante 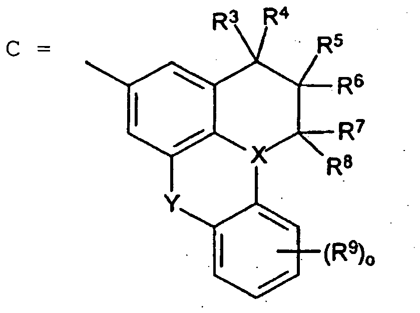
le radical R⁹, ainsi que les radicaux R¹ et R², étant définis, m, n et o représentant, indépendamment les uns des autres, un nombre entier entre 1 et 4 ;
les radicaux R³ jusqu'à R⁸ représentant indépendamment les uns des autres respectivement un substituant choisi dans le groupe α ; ou
un radical R³ ensemble avec un radical R⁴, ou un radical R⁵ ensemble avec un radical R⁶, ou un radical R⁷ ensemble avec un radical R⁸, indépendamment les uns des autres, forment un cycle carbo- ou hétéro-monocyclique de 3 à 8 chaînons avec leur atome de carbone commun respectif, qui porte éventuellement un ou plusieurs substituants faisant partie du groupe α, et sur lequel de un à trois systèmes cycliques aromatiques ou hétéro aromatiques peuvent être cyclisés, le, respectivement, les, système(s) cyclique(s) étant choisis indépendamment les uns des autres dans le groupe β, constitué par le benzène, le naphtalène, le phénanthrène, la pyridine, la quinoléine, le furane, le thiophène, le pyrrole, le benzofurane, le benzothiophène, l'indole et le carbazole, qui, à leur tour, peuvent être substitués avec un ou plusieurs substituants choisis dans le groupe α ; ou
quatre radicaux voisins R³ à R⁶, ou R⁵ à R⁸, forment un cycle benzylique ou pyridinique cyclisé sur le squelette de base non substitué, mono- ou disubstitué, dont les substituants peuvent être choisis dans le groupe α ;
X remplaçant N ou un groupe CR¹⁴, le radical R¹⁴ étant choisi dans le groupe constitué par de l'hydrogène, un groupe alkyle en C₁-C₆, et un groupe phényle non substitué, mono- ou disubstitué, dont les substituants sont choisis dans le groupe α ; et
Y remplaçant O, S, un groupe NR¹⁵, un groupe CR¹⁵R¹⁶, ou un groupe CR¹⁵R¹⁶-CR¹⁵R¹⁶, les radicaux R¹⁵ et R¹⁶ pouvant être choisis de la même manière que le radical R¹⁴, ou les deux radicaux R¹⁵ et R¹⁶ formant ensemble avec leur atome de carbone commun un cycle carbo- ou hétéro monocyclique de 3 à 8 chaînons, qui, éventuellement, porte un ou plusieurs substituants du groupe α, et sur lequel peuvent être cyclisés de un à trois systèmes cycliques aromatiques ou hétéro aromatiques, le, respectivement, les système(s) cyclique(s) étant choisi(s) indépendamment les uns des autres dans le groupe β, constitué par le benzène, le naphtalène, le phénanthrène, la pyridine, la quinoléine, le furane, le thiophène, le pyrrole, le benzofurane, le benzothiophène, l'indole et le carbazole, qui, à leur tour, peuvent être substitués avec un ou plusieurs substituants choisis dans le groupe α.

2. Naphtopyranes photochromes selon la revendication 1, qui présentent la formule (I).

3. Naphtopyranes photochromes selon les revendications 1 ou 2, qui présentent les formules générales (III), (IV) et (V) suivantes : 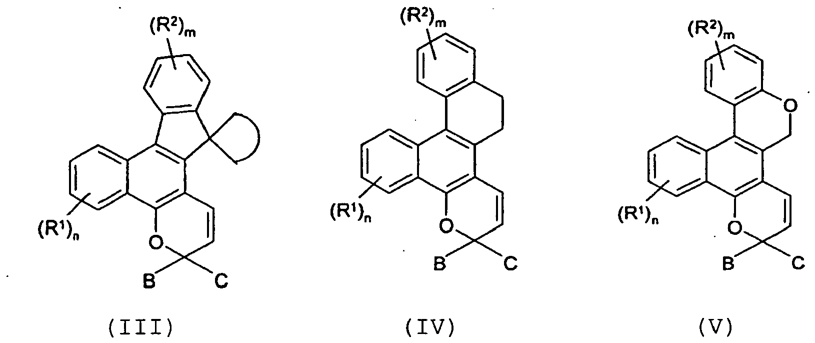 dans lesquelles tous les radicaux sont tels que définis ci-dessus et dans lesquelles le cycle spiro dans la formule (III) représente, moyennant l'incorporation de l'atome de carbone spiro, un cycle carbocyclique de 5 à 8 chaînons qui porte éventuellement un ou plusieurs substituants faisant partie du groupe α, et qui peuvent être cyclisés sur entre un et trois cycles benzéniques, qui, à leur tour, peuvent être substitués avec un ou plusieurs substituants choisis dans le groupe α.

4. Naphtopyranes photochromes selon l'une des revendications 1 à 3, le radical B étant choisi dans le groupe a).

5. Naphtopyranes photochromes selon l'une des revendications 1 à 4, X représentant N dans le radical C.

6. Naphtopyranes photochromes selon l'une des revendications 1 à 5, Y représentant le groupe CR¹⁵R¹⁶ ou le groupe CR¹⁵R¹⁶-CR¹⁵R¹⁶ dans le radical C, les radicaux R¹⁵, respectivement, R¹⁶, étant tels que définis ci-dessus.

7. Naphtopyranes photochromes selon l'une des revendications 1 à 6, les radicaux R³ à R⁸ représentant, indépendamment les uns des autres, respectivement, un substituant choisis dans le groupe α.

8. Naphtopyranes photochromes selon l'une des revendications 1 à 7, les radicaux R³ et R⁴ représentant indépendamment l'un de l'autre respectivement un substituant choisi dans le groupe α ; et simultanément, les quatre radicaux R⁵ à R⁸ formant ensemble un cycle commun benzénique ou pyridinique cyclisé sur le squelette de base non substitué, mono-ou disubstitué, dont les substituants peuvent être choisis dans le groupe α.

9. Naphtopyranes photochromes selon l'une des revendications 1 à 8, le radical C présentant les structures suivantes :

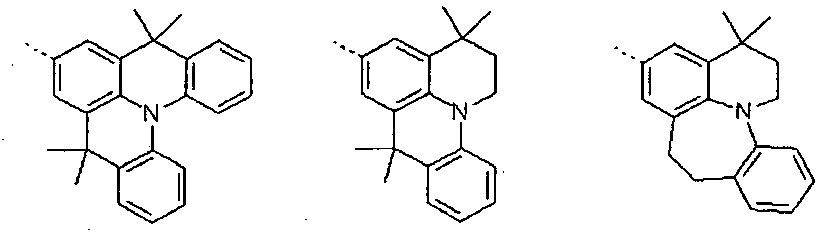

10. Naphtopyranes photochromes selon l'une des revendications 1 à 9, deux radicaux R² dans les formules précédentes (I) - (V) formant un cycle benzénique, pyridinique, benzofuranique ou benzothiénique cyclisé sur le squelette de base non substitué, mono- ou disubstitué, dont les substituants peuvent être choisis dans le groupe α.

11. Naphtopyranes photochromes selon l'une des revendications 1 à 10, deux radicaux R², se situant en position ortho l'un par rapport à l'autre, dans les formules (I) - (V) précédentes, représentant une cyclisation benzofuranique.

12. Naphtopyranes photochromes selon l'une des revendications 1 à 11, Z étant choisi parmi les groupes -CR¹⁰R¹¹-, -O-CR¹⁰R¹¹-, -CR¹⁰R¹¹-O-, -CR¹⁰R¹¹-CR¹⁰R¹¹ ou - CR¹⁰=CR¹¹-, les substituants R¹⁰ et R¹¹ étant choisis dans le groupe α.

13. Utilisation des naphtopyranes photochromes selon l'une des revendications 1 à 12 dans, et sur, des matériaux en substances synthétiques.

14. Utilisation selon la revendication 13, le matériau en substance synthétique étant une lentille ophtalmique.
